# EUROPÄISCHE PATENTSCHRIFT

(11) **EP 1 259 200 B1**
(45) Veröffentlichungstag und Bekanntmachung des Hinweises auf die Patenterteilung: **03.08.2005**
(21) Anmeldenummer: 01915291.7
(22) Anmeldetag: 23.02.2001
(51) Int. Cl.: A61F 2/46, B01F 13/00

(54) **AUFBEREITUNGS- UND APPLIKATIONSVORRICHTUNG FÜR IMPLANTATMATERIALIEN**
PREPARATION AND APPLICATION DEVICE FOR IMPLANT MATERIALS
DISPOSITIF DE PREPARATION ET D'APPLICATION DE MATERIAUX D'IMPLANT

(30) Priorität: 28.02.2000 DE 20003676 U; 22.03.2000 DE 20005333 U
(43) Veröffentlichungstag der Anmeldung: 27.11.2002
(73) Patentinhaber: Coripharm Medizinprodukte GmbH & Co. KG., 64807 Dieburg (DE)
(72) Erfinder: WAHLIG, Helmut, 64287 Darmstadt (DE); DINGELDEIN, Elvira, 63303 Dreieich (DE); SATTIG, Christoph, 64807 Dieburg (DE); WÜST, Edgar, 63110 Rodgau (DE)
(74) Vertreter: Helber, Friedrich G., Dipl.-Ing.
(86) Internationale Anmeldenummer: PCT/EP2001/002042
(87) Internationale Veröffentlichungsnummer: WO 2001/070146

(56) Entgegenhaltungen:
- EP-A- 0 402 669
- EP-A- 0 674 888
- EP-A- 0 692 229
- EP-A- 0 768 067
- EP-A- 0 796 653
- EP-A- 0 882 436
- WO-A-90/13264
- WO-A-97/18031
- DE-A- 2 801 706
- DE-A- 3 640 279
- DE-A- 4 228 780
- DE-A- 4 302 230
- US-A- 4 277 184
- US-A- 5 435 645

## Beschreibung

Die Erfindung betrifft eine Aufbereitungs- und Applikationsvorrichtung für aus wenigstens einer pulver- oder granulatförmigen und einer flüssigen Komponente unmittelbar vor Gebrauch aufzubereitende Implantatmaterialien mit einem ersten, die pulvrige oder granulatförmige Komponente steril abgeschlossen aufnehmenden Behälter und einem zweiten, die flüssige Komponente steril abgeschlossen aufnehmenden Behälter, welcher mit dem ersten Behälter verbindbar ist, wobei Mittel zur Herstellung einer Überströmverbindung zwischen den Behältern, zur Verdrängung der flüssigen Komponente aus dem zweiten in den ersten Behälter und zur innigen homogenen Vermischung der Komponenten im ersten Behälter durch Bewegung eines im ersten Behälter angeordneten, an einem abgedichtet aus dem Behälter herausgeführten hohlen Mischschaft angeordneten Mischorgans vorgesehen und der erste Behälter an eine Unterdruckquelle anschließbar ausgebildet ist und am ersten Behälter ein den im Behälter herrschenden Unterdruck anzeigendes Messgerät anschließbar ist

In der operativen Behandlung von Defekten am Skelettsystem haben sich in den letzten Jahren zunehmend minimal-invasive Behandlungsverfahren durchgesetzt. So wird insbesondere in der Orthopädie und Unfallchirurgie von Methoden Gebrauch gemacht, bei denen die Behandlung selbst, teilweise unter Röntgenkontrolle, z.B. nur noch durch Stichinzisionen oder perkutan durchgeführt wird. Der Vorteil dieser Verfahren gegenüber der konservativen, offenen Chirurgie liegt auf der Hand: Der Eingriff ist für den Patienten weit weniger belastend, die Liegezeiten im Krankenhaus werden signifikant verkürzt und damit die Behandlungskosten deutlich gesenkt. Außerdem ist die Infektionsgefahr wesentlich geringer, so dass langfristige und kostspielige Komplikationen vermieden werden können.

Speziell bei der rekonstruktiven Behandlung von Knochendefekten werden im Zuge der minimal-invasiven Behandlung Füll-, Stabilisierungs-, Hilfs- und/oder Wirkstoffe perkutan direkt an den Ort des Defektes gebracht. Sie dienen dort insbesondere der Defektauffüllung und Teilstabilisierung, sowie der Anregung von Reparaturvorgängen, der Induktion und Beschleunigung von Neovaskularisierung und Knochenneubildung, sowie der Verhütung und/oder Behandlung von Infektionen im Defektbereich. Die meisten der dabei zur Anwendung kommenden Materialien oder Wirkstoffe, bzw. deren Kombinationen, können allerdings aufgrund ihrer besonderen Zusammensetzung und der daraus resultierenden Konsistenz nicht mit herkömmlichen Injektionsspritzen appliziert werden. Vielmehr ist es notwendig, besondere, dem jeweiligen Produkt eigens angepasste und entsprechende Applikatoren anzuwenden, um die Therapeutika, d.h. die Implantatmaterialien, einfach und sicher an den Ort ihrer Anwendung zu bringen.
Selbstverständlich ist es notwendig, die meist aus verschiedenen Einzelkomponenten bestehenden Implantatmaterialien vor ihrer Applikation zusammenzuführen und zu einer homogenen Matrix zu vermischen.

Dabei hat sich allerdings gezeigt, dass die chemisch-physikalischen Eigenschaften der Implantate, insbesondere aber ihre mechanische Festigkeit - wichtige Voraussetzungen für die optimale Erfüllung ihrer Aufgaben - ganz wesentlich durch die Art und Weise des durchgeführten Mischvorganges beeinflusst werden.

Dies sei am Beispiel des Implantatmaterials Knochenzement, wo diese Einflüsse intensiv untersucht wurden, deutlich gemacht:
Seit mehreren Jahren ist bekannt, dass die mechanische Fertigkeit von Knochenzementen durch größere und kleinere Lufteinschlüsse, die insbesondere bei der Vermischung der Zementkomponenten in die resultierende Zementmatrix gelangen, erheblich reduziert wird. Diese Schwächung des Zementes ist physikalisch leicht mess- und nachweisbar und sie ist verständlich, wenn man berücksichtigt, dass, insbesondere bei unsachgemäßer Anmischung der Komponenten von Hand, bis zu 25 % Luft in der Zementmatrix enthalten sein können. Diese im Zement eingeschlossenen Luftblasen erzeugen Poren, die bei späterer Belastung durch die Prothese zu Riss- und Spaltenbildungen im Zement führen. Dies wiederum verursacht eine vorzeitige Zerrüttung des die Prothese umgebenden Zementmantels, was zu einer Prothesenlockerung mit der Notwendigkeit der Prothesenentfernung führt. Umgekehrt haben experimentelle und klinische Studien gezeigt, dass annähernd luft- und damit porenfreie Zemente zu einer höheren fatigue-Resistenz und damit zu einer Verlängerung der Lebensdauer von Endoprothesen beitragen.

Neben diesem Beispiel aus dem Bereich der Knochenzemente sei auf technische Verfahren bei der Keramikproduktion verwiesen. Auch dort ist es der Stand der Technik, insbesondere bei der Verarbeitung hochwertiger Gipse und Abformmaterialien porenfreie (d.h. insbesondere luftfreie) Matrizes herzustellen, um so die notwendigen mechanischen Festigkeiten des Endproduktes zu erreichen.

Sinngemäß müssen aber auch bei medizinischen Implantatmaterialien wie z.B. solche auf Basis von Kalziumphosphaten, Kalziumsulfaten oder gewisser Polymere, die neben ihrer physiologischen Wirkung auch noch eine stabilisierende Funktion auszuüben haben, die gleichen Voraussetzungen erfüllt sein. Auch bei der Zubereitung und Mischung dieser Produkte müssen Lufteinschlüsse sicher vermieden werden, um die gewünschte mechanische Festigkeit im Endprodukt des Implantatmaterials sicherzustellen.

Während allerdings im technischen Bereich bereits seit langem entsprechend verfahren wird, gibt es im medizinischen Bereich noch keine entsprechenden Anmisch- und/oder Applikationssysteme für z. B. keramische Implantatmaterialien. Bei den Knochenzementen hat die Erkenntnis dieser Problematik in den zurückliegenden Jahren zu einer intensiven Beschäftigung mit Anmischsystemen geführt, mit dem Ziel, Verfahren zu entwickeln, mit deren Hilfe es möglich ist, Lufteinschlüsse im Zement beim Anmischen der Zementkomponenten weitestgehend zu vermeiden, und Mischmethoden zu erarbeiten, die reproduzierbare und standardisierte Mischergebnisse sicherstellen.

Wiederum soll am Beispiel der sehr gut untersuchten Knochenzemente diese Entwicklung verdeutlicht werden: Das Ergebnis intensiver Bemühungen führte zu der heute allgemein anerkannten und als Stand der Technik angesehenen sogenannten "Vakuum-Mischtechnik", bei der das Mischen der Zementkomponenten (Polymerpulver und Monomerflüssigkeit) in besonders geeigneten und eigens hierfür gestalteten Anmischgefäßen bzw. Applikationskartuschen unter vermindertem Atmosphärendruck durchgeführt wird.

Um dabei ein "Vakuum" (einen reduzierten Luftdruck) zu erreichen, der den Luftgehalt in der fertig gemischten Zementmatrix auf ein Minimum reduziert, müssen während des Mischvorgangs Restdrucke von ca. 100 bis 200 mbar sichergestellt werden.

Praktisch wird hierfür das Zementanmischgefäß nach Einfüllen der Zementkomponenten dicht verschlossen und über einen Schlauch an eine mit Druckluft angetriebene Pumpe angeschlossen. Je nach Druckluftquelle und Pumpenkonstruktion wird dabei - mehr oder weniger schnell - die im Mischsystem enthaltene Luftmenge reduziert, so dass mehr oder weniger geringe Restdrucke resultieren, wobei es aber nicht mit jedem im Markt befindlichen System gelingt, den Luftdruck so weit abzusenken, dass tatsächlich das Ziel - ein annähernd porenfreier Zement - auch praktisch erreicht wird.

Ein ganz wesentliches Problem bei dieser Art der "Vakuum-Mischung" von Knochenzementen besteht darin, dass neben den sehr unterschiedlichen Pumpenleistungen insbesondere die zum Betreiben der Pumpen notwendige klinikeigene Druckluftversorgung im Operationsraum sehr großen Schwankungen unterworfen ist. So variiert die hauseigene Druckluft in verschiedenen Kliniken ggf. nicht nur tageszeitlich sondern oft generell zwischen Ausgangsdrucken von ca. 5 bis ca. 10 bar, was natürlich die Pumpenleistung und damit das "Vakuum" in den Anmischgefäßen und als Folge davon die Zementqualität bzgl. der Lufteinschlüsse erheblich beeinflusst. Standardisierbare und reproduzierbare Mischergebnisse sind auf diese Weise nicht zu realisieren.

Über dies haben nur einige der heute eingesetzten Pumpen ein Manometer, das die Pumpenleistung anzeigt und während des Mischvorganges kontrolliert. Diese Messeinrichtungen an den Pumpen selbst geben aber nicht notwendigerweise die Druckverhältnisse in den eigentlichen Anmischgefäßen wieder. Dabei ist eine sichere Kontrolle des Restdrucks im Anmischgefäß während der gesamten Dauer des Mischvorganges unbedingte Voraussetzung für ein adäquates, verlässliches Mischergebnis. Bei allen bekannten "Vakuum"-Mischsystemen fehlen tatsächlich geeignete Anzeige- oder Messeinrichtungen an den Mischgefäßen selbst, so dass nicht sichergestellt werden kann, dass in dem Anmischgefäß vor und/oder während des eigentlichen Mischvorgangs der Atmosphärendruck auch tatsächlich auf den für das Mischergebnis - nämlich ein weitestgehend porenfreier Zement - notwendigen Restdruck abgesenkt wurde.

Hieraus ergeben sich ganz wesentliche Nachteile für die zur Zeit angewandten Verfahren selbst und erhebliche Risiken für die Qualität des Zementes und damit verbunden für den klinischen Langzeiterfolg des künstlichen Gelenkersatzes.

Darüber hinaus gehen heute mehr und mehr Kliniken zu elektrisch angetriebenen chirurgischen Geräten über, so dass dann im OP überhaupt kein geeigneter Druckluftanschluss mehr zur Verfügung steht.

Um nun einerseits diese Fehlerquellen und Unwägbarkeiten auszuschließen, wie sie am Beispiel der "Vakuum-Anmischung" von Knochenzementen beschrieben werden, insbesondere aber auch, um andererseits für andere Implantatmaterialien, außer Knochenzementen, wie sie heute in der rekonstruktiven Chirurgie immer häufiger zur Anwendung kommen, die Herstellung porenfreier Matrizes in die Praxis umzusetzen, bestand die Aufgabe dieser Erfindung darin, ein vielseitig anwendbares, einfaches und sicher zu handhabendes Anmisch- und Applikationssystem zu schaffen, das dazu geeignet ist, die Zubereitung von in den menschlichen und/oder tierischen Körper zu verbringenden Implantatmaterialien im Hinblick auf ihre chemisch-physikalischen Eigenschaften zu optimieren. Wesentliche Indikationen für ein derartiges System finden sich insbesondere im Bereich der Orthopädie und Unfallchirurgie.

Bei der technischen Realisierung dieser Aufgabe war es also notwendig, zwei wesentliche Aspekte zu berücksichtigen.
1. Das erfindungsgemäße System soll die Herstellung weitgehend porenfreier Implantatmaterialien gewährleisten und außer für Polymere insbesondere auch für Keramikmaterialien im weitesten Sinne geeignet sein.
2. Das erfindungsgemäße System soll die einfache und sichere Zusammenführung der zur Herstellung des Implantatmaterials benötigten Komponenten ermöglichen. Der gesamte Vorgang der Komponenten-Zusammenführung und -Mischung soll unter sterilen Bedingungen und hermetischem Abschluss erfolgen, so dass kein Kontakt der Komponenten mit der Außenluft möglich ist.

Die Problematik der Herstellung porenfreier (d.h. von Lufteinschlüssen freier) Matrizes soll am Beispiel der Knochenzemente dargestellt werden. Diese Problematik ist allgemeingültig und trifft auch im wesentlichen auf die Mischung und Applikation andersartiger Implantatmaterialien zu. Allerdings ist sie im Bereich der Knochenzemente in den vergangenen Jahren eingehend untersucht und dokumentiert worden, weshalb auf diesen Sektor verwiesen wird. So haben - wie beschrieben, experimentelle und klinische Studien gezeigt, dass das herkömmliche Mischen der Zementkomponenten, z.B. unter Verwendung einfacher, schlüsselförmiger Gefäße und eines Spatels, nicht nur umständlich und aus aspetischer Sicht problematisch ist, sondern dass speziell bei dieser Technik sehr viel Luft in Form größerer und kleinerer Blasen in den Zementteig eingetragen wird. Diese Einschlüsse vermindern die mechanische Festigkeit des ausgehärteten Zementes, insbesondere seine "fatigue-resistance" erheblich und führen damit zu einem frühzeitigen Versagen der Prothesenfunktion.

Das sogenannte offene Mischen - wie beschrieben - hat aber auch noch weitere praktische Nachteile. Da die Verpackung der Zemente meist in einem Beutel für das Pulver und in einem Glasgefäß für die Flüssigkeit besteht, kann sehr leicht beim Ausschütten der Komponenten in das Anmischgefäß Pulver oder Flüssigkeit verschüttet werden. Dann ist aber die genau abgestimmte und für die einwandfreie Polymerisation notwendige Relation von Pulver zu Flüssigkeitsmenge nicht mehr gewährleistet, wodurch ebenfalls die mechanischen Eigenschaften des fertigen Zementes negativ beeinflusst werden können.

Weiterhin führt die offene Handhabung der Komponenten dazu, dass das Pulver beim Ausschütten eine starke Staubentwicklung bewirkt und dass aus der Flüssigkeit Monomerdämpfe in die Atemluft entweichen und zu einer Gesundheitsgefährdung - insbesondere zu allergischen Reaktionen - bei dem mit der Anmischung beschäftigen OP-Personal führen können. Viele der genannten Probleme treffen auch auf die Verarbeitung andersartiger Implantatmaterialien zu.

All diese negativen Erfahren haben dazu geführt, dass im Laufe der letzten Jahre Anmischverfahren erarbeitet wurden, die das Ziel hatten, die genannten Fehlerquellen zu vermeiden. So wurden Anmischgefäße entwickelt, bei denen der Behälter nach dem Einfüllen der Komponenten fest verschlossen wird, so dass während des Mischvorganges keine Monomerdämpfe entweichen können. Bei anderen Systemen erfolgt der Mischvorgang nach der Beschickung des Mischgefäßes unter reduziertem Atmosphärendruck. Dabei werden üblicherweise durch die Anwendung einer "Vakuumpumpe" die Monomerdämpfe abgesaugt und in einem Kohlefilter gebunden.

Neuerdings wurden nun Anmischsysteme bekannt, bei denen sich bereits vom Hersteller aus Polymerpulver und Monomerflüssigkeit in Teilen des Anmischsystems befinden. Dabei sind die beiden Komponenten zwar in getrennten Gefäßen abgefüllt, diese können jedoch miteinander verbunden werden. Auf diese Weise soll erreicht werden, dass der Mischvorgang ohne äußere Einflüsse oder Störungen durchgeführt werden kann. Beispiele für solche Systeme sind in den Anmeldungen US 4.277.184, US 5.328.262, US 5.549.380, US 5.551.778, DE 40 30 832 und EP 0 692 229 beschrieben. Aus der WO-A-97/18031 ist in diesem Zusammenhang auch eine Vorrichtung der eingangs erwähnten Art bekannt.

In der praktischen Herstellung und Anwendung verfügen aber auch diese Systeme noch über erhebliche Mängel und Nachteile. Insbesondere ist die sterile Herstellung solcher Packungseinheiten ein großes Problem. Grundsätzlich kommen für die Sterilisation von Knochenzementen nur drei Verfahren in Frage: Für das Monomer die Sterilfiltration und eine sterile Abfüllung. Für das Polymer die γ-Strahlen-Sterilisation oder die Äthylenbegasung.

Da der Behälter für das Polymerpulver in den bekannten Systemen zur Wahrung der Sterilität hermetisch abgeschlossen sein muss, werden diese Pulverbehälter üblicherweise zur Sterilisation einer γ-Bestrahlung unterzogen. Wie neuere Untersuchungen belegen (HARPER et al., J. of Mat. Sci.: Mat. in Med. 8, 849-853, 1997) führt aber die Behandlung des Zementpulvers mit energiereicher Strahlung zu einer erheblichen Verschlechterung der mechanischen Festigkeit des Zementes und zu einem erhöhten Risiko für vorzeitige mechanischen Prothesenlockerungen. Natürlich kann auch ein Mischsystem, bei dem die Behälter für Polymer und Monomer miteinander verbunden sind, keiner γ-Bestrahlung unterzogen werden, weil dies zu einer vorzeitigen Auspolymerisation des Monomers führen würde.

Grundsätzlich wäre aber ein System, das die geschilderten Nachteile der bestehenden Vorrichtungen sicher vermeidet und darüber hinaus insbesondere für die heute immer mehr an Bedeutung gewinnenden, vorwiegend keramischen Implantatmaterialien (außer Knochenzemente), für die ebenfalls die porenfreie Herstellung von entscheidender Bedeutung ist, von großem Nutzen, da sie die Handhabung der Implantatmaterialien im OP ganz wesentlich vereinfachen, insbesondere aber auch in jeder Hinsicht sicherer machen würde.

Es besteht somit ein Bedürfnis, eine Problemlösung zu finden, die für die verschiedenartigsten, heute in Anwendung befindlichen Implantatmaterialien universell geeignet ist und bei der einerseits die Vorteile, die die Bereitstellung hermetisch abgeschlossener, vorab eingefüllter, steriler Komponenten bietet genutzt werden, die andererseits aber die bei herkömmlichen Systemen bestehenden Nachteile und klinischen Sicherheitsrisiken sicher vermeidet, wobei insbesondere ein Anmischsystem von Vorteil wäre, das von äußeren Bedingungen und Einflüssen völlig frei ist und absolut unabhängig von den klinischen Voraussetzungen bzgl. des Vorhandenseins einer nach Menge pro Minute und Höhe des Ausgangsdruckes geeigneten Druckluftversorgung oder einer elektrischen Energiequelle betrieben werden kann. Darüber hinaus erscheint es zweckmäßig, die "Vakuumquelle" unmittelbar in das Mischsystem zu integrieren, um so eine einfache und sichere Handhabbarkeit des Mischens zu gewährleisten und insbesondere frei von lästigen und den Betrieb des Systems störenden Schlauchverbindungen zu sein, da sich gezeigt hat, dass hierdurch in der Praxis immer wieder Pannen entstehen und die herkömmlichen Pumpen-Systeme unhandlich und schwerfällig zu bedienen und nur aufwendig zu sterilisieren und zu warten sind. Außerdem sollte das neue Mischsystem so gestaltet sein, dass während des gesamten Mischvorgangs eine kontinuierliche Kontrolle des in dem Mischgefäß selbst herrschenden Restdruckes möglich ist.

Im Rahmen der, aus vielerlei Gründen sehr vorteilhaften und heute mehr und mehr bevorzugten minimal-invasiven Verfahren bei der Behandlung von Knochendefekten, werden - wie beschrieben - die zur Anwendung kommenden Implantatmateralien bevorzugt lokal, direkt am Ort des Defektes, eingesetzt.

Dabei hat sich jedoch gezeigt, dass insbesondere bei der Behandlung von Knochendefekten, wie z.B. Frakturen, Pseudoarthrosen und Zysten, die Injektion flüssiger Implantatmaterialien und/oder Wirkstoffe ungeeignet und wenig sinnvoll ist. Vielmehr muss erreicht werden, dass die Implantate in einer Konsistenz appliziert werden, die es sicher stellt, dass sie lokal, am Zielort, für längere Zeit ortsfest verbleiben und so die biologischen Rekonstruktionsabläufe einleiten können. Außerdem müssen dabei Implantatmaterialien berücksichtigt werden, die sich vor Ort verfestigen, gewisse Stütz- und Stabilisierungsfunktionen zu erfüllen haben und auch als Wirkstoffträger dienen können.

Dieses Ziel ist in besonders vorteilhafter Weise dadurch zu erreichen, dass die Implantatmaterialien entweder in Form einer Paste, oder dass Wirkstoffe als Bestandteil einer viskösen Matrix verabfolgt werden, wobei es besonders günstig ist, wenn sich die Matrix vor Ort innerhalb einer gewissen, nicht zu langen Zeit verfestigt und so das Implantat, ggf. bestehend aus Trägermaterialien und Wirkstoffen, für längere Zeit am Ort des Defektes zur Verfügung steht. Wünschenswert ist es dabei allerdings, dass die applizierte Matrix abbaubar und/oder absorbierbar ist, so dass, nach einem gewissen Zeitraum, die zunächst von der Matrix ausgefüllten und stabilisierten Defekte von neugebildetem Knochen durchbaut sind und damit die ursprüngliche physiologische und biologische Beschaffenheit des betroffenen Skelettabschnittes wieder in vollem Umfang hergestellt und die volle Belastbarkeit gewährleistet ist.

Derartige pastöse oder visköse, ggf. sich lokal verfestigende Implantate bestehen notwendigerweise sehr oft aus zwei oder mehr Komponenten, die zunächst getrennt sind und erst nach dem Zusammenführen und Mischen appliziert werden können. Hierbei bestehen solche Systeme meistens aus einer trockenen, pulverförmigen Komponente und einer zweiten flüssigen Komponente. Bei den Pulvern kann es sich dabei um Einzelbestandteile oder Kombinationen aus z.B. Hydroxylapatit, Trikalziumphosphat, Kalziumsulfat, Kalziumkarbonat, Polymere, insbesondere solche auf Basis von Polyacrylaten und/oder Polymethacrylaten, weiterhin Lactid, Glykolid und/oder Lactid/Glykolid, Kollagen, Polysaccharide wie Agarose, Gelatine, Fibrin oder dergleichen handeln. Bei den korrespondierenden Flüssigkeiten sind insbesondere solche auf wässriger Basis (wie z.B. Wasser (aqua pro injectione), Pufferlösungen, Ringerlösung, physiologische Kochsalzlösung, Blut, Serum usw.) oder geeignete organische Flüssigkeiten wie z.B. Monomerflüssigkeiten zu nennen. Selbstverständlich ist es notwendig, die Einzelkomponenten vor ihrer Applikation zusammenzuführen und zu einer homogenen, porenfreien Matrix zu vermischen. Für diesen Vorgang können herkömmliche Mischverfahren, wie sie z.B. aus dem Bereich der Knochenzemente oder von der Mischung und Verarbeitung von Keramikwerkstoffen bekannt sind, Verwendung finden.

Für die Handhabung solcher Systeme, einschließlich der sterilen, einfachen und sicheren lokalen Applikation des Implantatmaterials unmittelbar nach seiner Anmischung, ist es sehr vorteilhaft, einerseits die sterile Pulverkomponente in einem, ggf. als Mischsystem ausgestatteten, hermetisch verschlossenen Gefäß bereit zu stellen und andererseits auch die sterile Flüssigkeit in einem besonderen Gefäß, hermetisch verschlossen, zur Verfügung zu haben. Beide Gefäße müssen in einer sterilen Doppelverpackung, wie sie für im OP benutzte Materialien notwendig ist, vorliegen. Nach der Entnahme der Gefäße aus der Verpackung müssen dann unter sterilen Bedingungen die in den beiden Behältern enthaltenen Komponenten vereint, gemischt und appliziert werden.

Ausgehend von den vorstehenden Ausführungen und den geschilderten klinisch-therapeutischen Problemen und Anforderungen besteht die Aufgabe der vorliegenden Erfindung darin, eine einfache und sichere Lösung für das Zusammenführen von Implantat-Komponenten zu finden, sowie das Problem zu lösen, die "Vakuum"-, Misch- und Vorratseinheiten so zu gestalten, dass das gesamte System gleichzeitig auch zur homogenen Mischung des Inhaltes ggf. unter reduziertem Atmosphärendruck und zur anschließenden Applikation der Mischung in den Knochendefekt geeignet ist. Insbesondere soll der Flüssigkeitsinjektor (siehe unten) für den Fall, dass er organische Lösungsmittel, etwa ein Monomer, wie z.B. Methylacrylat / Methylmethacrylat, enthalten soll, so gestaltet sein, dass er absolut dicht ist, um auch über längere Zeit eine Lagerstabilität des Monomeren ohne Substanzverlust sicher zustellen, was aber erfahrungsgemäss nicht über einen Kolben erreicht werden kann. Der Flüssigkeitsinjektor sollte darüber hinaus auch für die Aufnahme visköser Materialien oder Suspensionen geeignet sein.

Ausgehend von einer Vorrichtung der eingangs erwähnten Art wird diese Aufgabe erfindungsgemäß dadurch gelöst, dass der hohle Mischschaft an seinem äußeren Ende an eine Handpumpe zur Erzeugung des Unterdrucks angeschlossen und mit der Handpumpe verbunden ist dass der hohle Mischschaft in der ganz eingeschobenen Stellung in seinem abgedichtet durch die Behälterwandung geführten Bereich mit einer Durchgangsöffnung versehen ist, die mit einem in diesem Bereich mit einem selbstschließenden Ventil versehenen und ins Behälterinnere geführten Durchlasskanal verbunden ist und dass das Gehäuse der Handpumpe als Handhabe zur Bewegung des Mischschafts geeinet ausgebildet ist.

Durch diese Ausgestaltung wird erreicht, dass die in den separaten hermetisch abgeschlossenen Behältern enthaltenen sterilen Inhaltsstoffe unter sterilen Kautelen vereint werden können, ohne dass dabei der Inhalt der Behälter mit der Außenluft in Berührung gelangen kann. Dies wird insbesondere durch das verwendete Verbindungsmodul, d.h. den Perforationszylinder, erreicht, welcher bei der Zusammenfügung des Pulvergefäßes und des Flüssigkeitsinjektors gleichzeitig die die Behälter hermetisch verschließenden Folien oder Membranen perforiert und nach Abschluss des Mischvorganges leicht zu entfernen ist. An seiner Stelle kann dann eine Injektionstülle für die einfache und sichere lokale Applikation des fertiggestellten Implantatmaterials angebracht werden.

Überraschenderweise hat sich dabei gezeigt, dass die erfindungsgemäße Vorrichtung in der Handhabung sehr viel einfacher und sicherer als bekannte Mischgeräte ist. Es wurde aber auch gefunden, dass die auf einem einfachen Baukastensystem beruhende Erfindung geeignet ist, Mischgefäße, die möglicherweise mit verschiedenartigen Implantatmaterialien gefüllt sind, wahlweise und den jeweiligen konkreten, praktischen klinischen Anforderungen entsprechend zu kombinieren. Diese Möglichkeit ist sehr vorteilhaft und bietet dem anwendenden Kliniker ganz neue therapeutische Möglichkeiten.

Vorteilhafte Weiterbildungen der erfindungsgemäßen Vorrichtung sind in den Unteransprüchen beansprucht.

Die Erfindung ist in der folgenden Beschreibung eines Ausführungsbeispiels in Verbindung mit der Zeichnung näher erläutert, und zwar zeigt:
- Fig. 1: eine teilweise entlang der Längsmittelebene geschnittene und teilweise zusätzlich aufgebrochene Ansicht der erfindungsgemäßen Aufbereitungs- und Applikationsvorrichtung; und
- Fig. 2: eine in Fig. 1 innerhalb des strichpunktierten Ovals 2 liegende Einzelheit im Längsmittelschnitt, welche die abgedichtete Führung des Mischschafts in der Behälter-Verschlusskappe veranschaulicht.

Die in ihrer Gesamtheit mit 10 bezeichnete erfindungsgemäße Aufbereitungs- und Applikationsvorrichtung vereint verschiedene Konstruktionseinheiten und besteht im wesentlichen aus einem Gefäß, dem Pulverbehälter A, zur Aufnahme eines beliebigen Implantatmaterials in Form eines Pulvers oder Granulates, ggf. in Kombination mit einem oder mehreren pharmazeutischen Wirkstoffen, einer integrierten Vakuum-Pumpe B, einem Perforationszylinder C, einem Flüssigkeitsinjektor D, der die Anmischflüssigkeit enthält und einem Führungsbecher E.

### Zu A:

Der Behälter 12 für die Aufnahme der Trockensubstanz ("Pulverbehälter") wird mit eingefülltem Implantatmaterial (trockene Komponenten, vorzugsweise in Form feiner Pulver und/oder Granulate) und eingeschraubtem Perforationszylinder 14 dem Anwender zur Verfügung gestellt.

Der Pulverbehälter ist an einem Ende durch eine Verschlusskappe 16 verschlossen. Die gegenüberliegende Öffnung wird durch eine in den Perforationszylinder 14 fest integrierte Lamelle 18 verschlossen. An die Verschlusskappe ist ein in dem Pulverbehälter 12 in axialer Richtung verschieblicher Kolben 20 eingelassen, sowie eine Filterscheibe 22, ein Ventil 24 und ein Manometer 26 zur Messung des Unterdrucks im Pulverbehälter während des Mischvorgangs integriert.

Die Verschlusskappe 16 besitzt eine zentrische, axiale Öffnung, durch die passgenau und dichtend ein Mischschaft 28 mit Mischpaddel 30 und Sollbruchstelle 32 geführt ist.

### Zu B:

Auf den Mischschaft 28 ist in axialer Verlängerung die Vakuum-Handpumpe 34 aufgesetzt. Diese besteht aus dem Pumpengehäuse 36 und einer Gehäusestirnwand 38. Durch eine zentrische, axiale Öffnung in der Gehäusestimwand 38 ist ein Pumpenschaft 40 mit Handgriff 42 und Pumpenkolben 44 geführt. Im dem Behälter zugewandten Boden des Pumpengehäuses befindet sich ein Ventil 46.

### Zu C:

Der Perforationszylinder 14 trägt in seiner zentralen, zylindrischen Öffnung einen in axialer Richtung verschieblichen zylindrischen scheibenförmigen Körper 48, in den zentrisch eine an beiden Enden zugespitzte Perforationskanüle 50 integriert ist, sowie eine scheibenförmige Handhabe 52.

### Zu D:

Der Flüssigkeitsinjektor 54, zur Aufnahme der flüssigen Implantat-Komponenten wird mit eingefüllter Anmischflüssigkeit dem Anwender zur Verfügung gestellt. An einem Ende verjüngt er sich zu einem zylindrischen Stutzen 56, der mittels einer Folie oder Membran 58 verschlossen ist. Die gegenüberliegende Öffnung ist ebenfalls mittels einer Folie oder Membran 60 verschlossen. Im Flüssigkeitsinjektor befindet sich ein in axialer Richtung beweglicher Kolben 62 mit Dichtungslippen 64 und einem zentralen Stutzen 66, der eine zylindrische Öffnung 68 besitzt.

### Zu E:

Der zylindrische Führungsbecher 70 dient der Aufnahme und Führung des Flüssigkeitsinjektors. Im Boden des Führungsbechers sind drei Schubstangen 72 eingelassen, die an den freien Enden 74 zugespitzt sind und im Querschnitt ein Kreuzprofil besitzen.

Zum Gebrauch der Vorrichtung ist der Mischschaft 28 mit dem als Mischpaddel 30 ausgebildeten Mischorgan zunächst ganz in den Pulverbehälter 12 eingeschoben. Der Flüssigkeitsinjektor 54 wird mit seinem Stutzen 56 in die zylindrische Öffnung des in den Behälter 12 eingeschraubten Perforationszylinders 14 eingepresst. Dabei wird die Membran 58 des Stutzens 56 durch das eine Ende der Perforationskanüle 50 durchbrochen. Gleichzeitig schiebt der Stutzen 56 des Flüssigkeitsinjektors 54 den Scheibenkolben 48 des Perforationszylinders 14 in Richtung auf den Pulverbehälter 12 vor, wobei das andere Ende der Perforationskanüle 50 die Lamelle 18 im Perforationszylinder durchbricht. Jetzt besteht eine durchgängige Verbindung zwischen Pulverbehälter 12 und Flüssigkeitsinjektor 54. Nun wird der Flüssigkeitsinjektor in axialer Richtung in den Führungsbecher eingeschoben und dabei gegen die Spitzen 74 der Schubstangen 72 gedrückt. Diese durchbrechen die Folie 60 des Flüssigkeitsinjektors 54 und drücken ihrerseits den Kolben 62 in Richtung auf den Pulverbehälter 12, wodurch die Flüssigkeit aus dem Flüssigkeitsinjektor 54 durch die Perforationskanüle 50 des Perforationszylinders 14 in den Mischraum des Pulverbehälters 12 injiziert wird.

Jetzt wird der Pumpenschaft 40 der Vakuum-Handpumpe 34 mittels des Handgriffs 42 auf- und abbewegt. Dabei wird durch die Arbeit des Pumpenkolbens 44 über die beiden Ventile 24, 46, die sich entsprechend des Wegs des Pumpenkolbens öffnen bzw. schließen, die Luft aus dem Pulverbehälter entfernt. Der dabei im Pulverbehälter erzeugte Unterdruck kann mittels des Manometers 26 während des gesamten Mischvorgangs gemessen und kontrolliert werden.

Sobald der gewünschte Unterdruck im System erreicht ist, wird nun das Pumpengehäuse 36 mit ganz eingeschobenem Pumpenschaft 40 als Handgriff für die Betätigung des Mischschaftes 28 mit Mischpaddel 30 benutzt. Durch drehende Auf- und Abbewegung des Mischpaddels 30 werden im Pulverbehälter 12 die trocknen und flüssigen Komponenten des Implantationsmaterials für eine vorgegebene Zeit homogen gemischt. Infolge des Unterdrucks im System werden dabei Lufteinschlüsse aus dem Implantationsmaterial entfernt.

Nach Beendigung des Mischvorgangs wird der Mischschaft 28 ganz herausgezogen, bis das Mischpaddel an die Verschlusskappe 16 anschlägt. Dann wird der Mischschaft über der Verschlusskappe an der Sollbruchstelle 32 abgebrochen.

Nun wird der Flüssigkeitsinjektor 54 mitsamt dem Perforationszylinder mittels der Handhabe 52 aus dem Pulverbehälter 12 ausgeschraubt. Anstell der so entfernten Teile kann nun eine Tülle ("Schnorchel") in die Öffnung des Pulverbehälters eingeschraubt und dieser in eine übliche Applikationspistole eingelegt werden. Durch Niederdrücken des Kolbens 20 mittels der Applikationspistole lässt sich dann das Implantationsmaterial aus dem Pulverbehälter ausdrücken und über die Tülle direkt in das knöcherne Implantatlager applizieren.

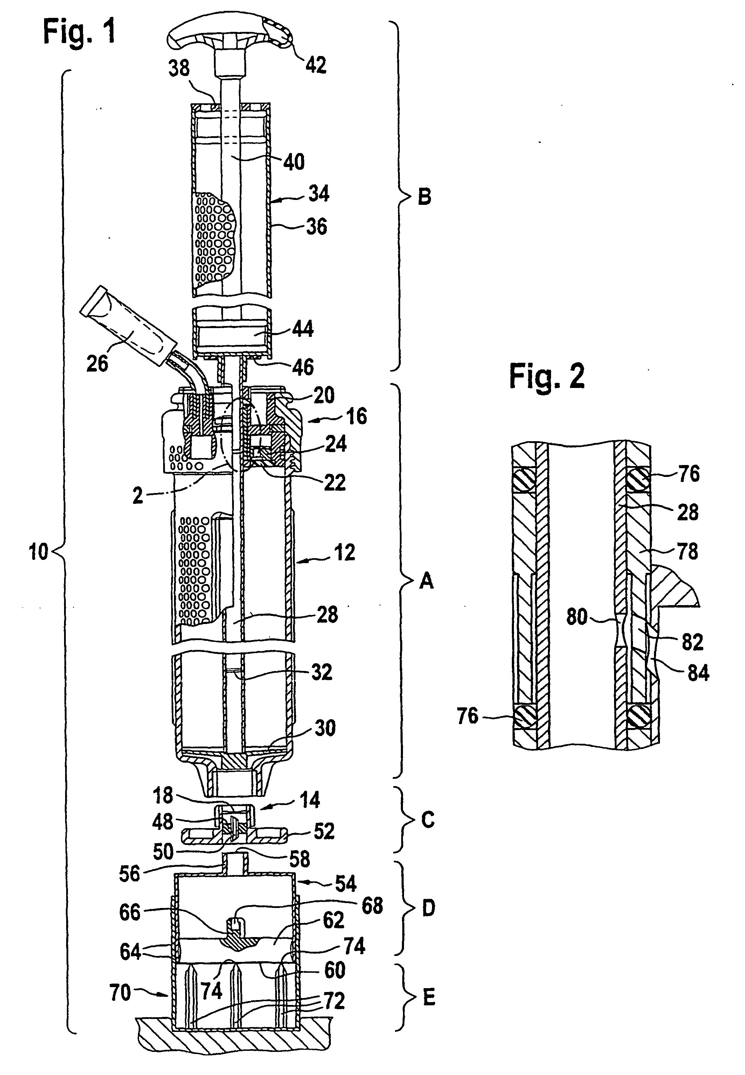

In Fig. 2 ist die Abdichtung des Mischschafts 28 in der Verschlusskappe 16 bzw. dem in der Verschlusskappe vorgesehenen Kolben 20 veranschaulicht. Es ist ersichtlich, dass diese Abdichtung durch zwei in Längsrichtung voneinander beabstandete O-Ringe 76 erfolgt, welche zwischen einer den Mischschaft 28 umgebenden Hülse 78 angeordnet sind. Eine Durchgangsöffnung 80 im Mischschaft 28 steht in der in Fig. 1 gezeigten ganz eingeschobenen Stellung des Mischpaddels 30 in den Behälter der Hülse 78 gegenüber, die ihrerseits mit einer Durchgangsöffnung 82 versehen ist, welche in einen im Behälterinnern mündenden und dem selbstschließenden Ventil 24 versehenen Durchlasskanal 84 mündet. Es ist also ersichtlich, dass beim Betätigen der Handpumpe 34 durch Hochziehen des Kolbens 44 das unter dem Kolben entstehende Vakuum über das hohle Innere des Mischschafts 28, die Durchlassöffnungen 80 und 82 und den Durchlasskanal 84 im Behälterinnern wirksam wird und den dort herrschenden Druck absenkt. Infolge des Zusammenwirkens der Ventile 24 im Kolben und 46 in der Pumpe 34 wird sichergestellt, dass beim Abwärtshub des Kolbens 44 im Gehäuse 36 der Pumpe 34 gegebenenfalls entstehender Überdruck das Ventil 24 schließt und über das Ventil 46 zur Atmosphäre abgeblasen wird. Der im Behälter 12 erzeugte Unterdruck kann dann durch nachfolgende Betätigung der Handpumpe 34 weiter abgesenkt werden.

## Patentansprüche

1. Aufbereitungs- und Applikationsvorrichtung (10) für aus wenigstens einer pulver- oder granulatförmigen und einer flüssigen Komponente unmittelbar vor Gebrauch aufzubereitende Implantatmaterialien mit einem ersten, die pulvrige oder granulatförmige Komponente steril abgeschlossen aufnehmenden Behälter (12) und einem zweiten, die flüssige Komponente steril abgeschlossen aufnehmenden Behälter (54), welcher mit dem ersten Behälter (12) verbindbar ist, wobei Mittel zur Herstellung einer Überströmverbindung zwischen den Behältern, zur Verdrängung der flüssigen Komponente aus dem zweiten in den ersten Behälter (12) und zur innigen homogenen Vermischung der Komponenten im ersten Behälter durch Bewegung eines im ersten Behälter angeordneten, an einem abgedichtet aus dem Behälter herausgeführten hohlen Mischschaft (28) angeordneten Mischorgans (30) vorgesehen und der erste Behälter an eine Unterdruckquelle anschließbar ausgebildet und am ersten Behälter ein den im Behälter herrschenden Unterdruck anzeigendes Messgerät anschließbar ist,
**dadurch gekennzeichnet,**
**dass** der hohle Mischschaft (28) an seinem äußeren Ende an eine Handpumpe (34) zur Erzeugung des Unterdrucks angeschlossen und mit der Handpumpe (34) verbunden ist,
**dass** der hohle Mischschaft (28) in der ganz eingeschobenen Stellung in seinem abgedichtet durch die Behälterwandung geführten Bereich mit einer Durchgangsöffnung (80) versehen ist, die mit einem in diesem Bereich mit einem selbstschließenden Ventil (24) versehenen und ins Behälterinnere geführten Durchlasskanal (84) verbunden ist, und
**dass** das Gehäuse (36) der Handpumpe (34) als Handhabe zur Bewegung des Mischschafts (28) geeignet ausgebildet ist.

2. Vorrichtung nach Anspruch 1, **dadurch gekennzeichnet, dass** zur Verbindung des ersten Behälters (12) mit dem zweiten Behälter (54) ein Perforationszylinder (14) vorgesehen ist, der in seinem dem ersten Behälter (12) zugewandten Endbereich durch eine Lamelle (18) dicht verschlossen und in einer Aufnahme des ersten Behälters (12) einführ- und dort befestigbar ist, und in den ein vom zweiten Behälter (54) vortretender durch eine Membran (58) dicht verschlossener Stutzen (56) einführbar ist, dass im Innern des Perforationszylinders (14) ein scheibenförmiger Körper (48) in Längsrichtung des Perforationszylinders (14) verschieblich angeordnet ist, welcher von einer hohlen, beidseitig vorstehenden und an beiden Enden zugeschärften Perforationskanüle (50) durchsetzt wird, und dass die Länge des vom zweiten Behälter (54) vortretenden Stutzens (56) so bemessen ist, dass dieser beim Einführen in den in der Aufnahme des ersten Behälters (12) befestigten Perforationszylinder (14) den scheibenförmigen Körper (48) in Richtung des ersten Behälters (12) verschiebt, wobei sowohl die den Perforationszylinder (14) verschließende Lamelle (18) als auch die den Stutzen (56) des zweiten Behälters (54) verschließende Membran (38)von der Perforationskanüle (50) durchstochen wird.

3. Vorrichtung nach Anspruch 2, **dadurch gekennzeichnet, dass** der zweite Behälter (54) an seinem dem Stutzen (56) gegenüber liegenden Ende durch eine Membran (60) dicht verschlossen ist, an welche im Behälterinnern anschließend ein verschieblicher, an der Innenwandung des Behälters (54) abgedichteter Kolben (62) angeordnet ist, und dass der zweite Behälter (54) in einem Führungsbecher (70) verschieblich gehaltert ist, von dessen geschlossenem Boden an ihren freien Enden (74) zugespitzte Schubstangen (72) vortreten, welche beim Verschieben des Führungsbechers (70) in Richtung auf den Stutzen (56) des zweiten Behälters (54) die den zweiten Behälter bodenseitig dicht verschließende Membran (60) durchstoßen und den anschließenden Kolben (62) in Richtung zum Stutzen (56) verschieben.

4. Vorrichtung nach einem der Ansprüche 1 bis 3, **dadurch gekennzeichnet, dass** der abgedichtet und verschieblich durch eine den ersten Behälter (12) abschließende Verschlusskappe geführte Mischschaft (28) mit einer Sollbruchstelle (36) versehen ist, welche in der ganz aus dem ersten Behälter herausgezogenen Stellung des Mischschafts (28) außerhalb des Behälters im Bereich der Verschlusskappe (16) angeordnet ist.

5. Vorrichtung nach Anspruch 4, **dadurch gekennzeichnet, dass** die Verschlusskappe (16) von einem an der Innenwandung des ersten Behältes abdichtend anliegenden verschieblichen Kolben (20) durchsetzt wird, dessen dem Behälterinnern abgewandte Stirnfläche die Verschlusskappe (16) in der Ausgangsstellung etwa bündig abschließt, durch Ausübung einer ins Behälterinnern gerichteten Kraft jedoch in Richtung der Aufnahme für den Perforationszylinder (14) verschieblich ist.

6. Vorrichtung nach einem der Ansprüche 1 bis 5, **dadurch gekennzeichnet, dass** die am äußeren Ende des Mischschafts (28) angeordnete Handpumpe (34) zur Erzeugung des Unterdrucks im ersten Behälter (12) als Kolbenpumpe mit einem zylindrischen Gehäuse (36) angeordneten Kolben (44) ausgebildet ist, der von einem aus der dem ersten Behälter abgewandten Gehäuse-Stirnwand (38) herausgeführten Pumpenschaft (40) mit an dessen freiem Ende vorgesehener Handhabe (42) im Gehäuse verschieblich ist.

7. Vorrichtung nach Anspruch 6, **dadurch gekennzeichnet, dass** in der mit dem Mischschaft (28) verbundenen Stirnwand des Pumpengehäuses (36) ein bei behälterseitig in der Handpumpe durch Verschiebung des Pumpenkolbens (44) erzeugtem Unterdruck selbstschließendes und bei auftretendem Überdruck öffnendes Ventil (46) vorgesehen ist.

8. Vorrichtung nach einem der Ansprüche 2 bis 7, **dadurch gekennzeichnet, dass** die Aufnahme für den Perforationszylinder (14) mit einem Innengewinde und der Perforationszylinder (14) mit einem komplementären Außengewinde versehen ist.

9. Vorrichtung nach Anspruch 8, **dadurch gekennzeichnet, dass** der Perforationszylinder (14) mit einer im Durchmesser vergrößerten Handhabe (52) versehen ist.

## Claims

1. Preparation and application device (10) for implant materials which are composed of at least one component in powder or granular form and a liquid component and are to be prepared immediately before use, with a first container (12) which receives the component in powder or granular form so as to be closed off in a sterile manner and a second container (54) which receives the liquid component so as to be closed off in a sterile manner and which can be connected to the first container (12), wherein means are provided for the production of an overflow connection between the containers, for the displacing of the liquid component from the second into the first container (12) and for the intimate homogeneous mixing of the components in the first container by movement of a mixing device (30) which is disposed in the first container on a hollow mixing shaft (28) which is guided in a sealed manner out of the container, and wherein the first container is constructed so that it can be connected to a negative pressure source and a measuring device can be connected to the first container to indicate the negative pressure prevailing in the container, **characterised in that** the hollow mixing shaft (28) is joined at its outer end to a hand pump 34 in order to produce the negative pressure and connected to the hand pump, that the hollow mixing shaft (28) in the fully inserted position is provided in its region which is guided in a sealed manner through the container wall with a through opening (80) which is connected to a through channel (84) which is provided in this region with a self-closing valve (24) and extends into the interior of the container, and that the housing (36) of the hand pump (34) is suitably constructed as a handle for movement of the mixing shaft (28).

2. Device as claimed in Claim 1, **characterised in that** in order to connect the first container (12) to the second container (54) a perforating cylinder (14) is provided which is closed and sealed in the end region thereof facing the first container (12) by a disc (18) and which can be inserted and fixed in a receptacle in the first container (12), and into which a stub which projects from the second container (54) and is closed and sealed by a membrane (58) can be inserted, that in the interior of the perforating cylinder (14) a disc-shaped body (48) is disposed so as to be displaceable in the longitudinal direction of the perforating cylinder (14) and has passing through it a hollow perforating cannula (50) which projects on both sides and is pointed at both ends, and that the length of the stub (56) projecting from the second container (54) is dimensioned so that the stub (56), when it is inserted into the perforating cylinder (14) which is fixed in the receptacle of the first container (12), displaces the disc-shaped body (48) in the direction of the first container (12), wherein both the disc (18) which closes the perforating cylinder (14) and also the membrane (38) which closes the stub (56) of the second container (54) are pierced by the perforating cannula (50).

3. Device as claimed in Claim 2, **characterised in that** the second container (54) is closed and sealed on its end lying opposite the nozzle (56) by a membrane (60) after which there is disposed in the interior of the container a movable piston (62) which is sealed against the inner wall of the container (54), and that the second container (54) is displaceably retained in a guide cup (70) which has projecting from its closed base push rods (72) which are pointed at their free ends (74) and which, when the guide cup (70) is displaced in the direction of the stub (56) of the second container (54), penetrate the membrane (60) which closes and seals the base of the second container and displace the following piston (62) in the direction of the stub (56).

4. Device as claimed in any one of Claims 1 to 3, **characterised in that** the mixing shaft (28), which is displaceably guided in a sealed manner through a sealing cap which closes off the first container (12), is provided with a predetermined breaking point (36) which is disposed outside the container in the region of the closure cap (16) when the mixing shaft (28) is completely withdrawn from the first container.

5. Device as claimed in Claim 4, **characterised in that** the closure cap (16) is penetrated by a displaceable piston (20) which rests in a sealing manner on the inner wall of the first container and of which the end face remote from the interior of the container closes off the closure cap (16) approximately flush in the initial position but is displaceable in the direction of the receptacle for the perforating cylinder (14) by exertion of a force directed into the interior of the container.

6. Device as claimed in one of Claims 1 to 5, **characterised in that** the hand pump (34) disposed on the outer end of the mixing shaft (28) for generating the negative pressure in the first container (12) is constructed as a piston pump having a piston (44) which is disposed in a cylindrical housing (36) and is displaceable in the housing by a pump shaft (40) which is guided out of the housing end face (38) remote from the first container and has a handle (42) provided on its free end.

7. Device as claimed in Claim 6, **characterised in that** a valve (46), which is self-closing when a negative pressure is generated on the container side in the hand-operated pump by displacement of the pump piston (44) and which opens when an overpressure occurs, is provided in the end face of the pump housing (36) which is connected to the mixing shaft (28).

8. Device as claimed in any one of Claims 2 to 7, **characterised in that** the receptacle for the perforating cylinder (14) is provided with an internal thread and the perforating cylinder (14) is provided with a complementary external thread.

9. Device as claimed in Claim 8, **characterised in that** the perforating cylinder (14) is provided with a handle (52) with an enlarged diameter.

## Revendications

1. Dispositif de préparation et d'administration (10) de matériaux pour implant, contenant au moins un composant en poudre ou en granulés et un composant liquide et destinés à être préparés directement avant l'utilisation, comportant un premier conteneur (12) fermé de manière stérile et contenant le composant en poudre ou en granulés et un deuxième conteneur (54) fermé de manière stérile et contenant le composant liquide, lequel peut être relié au premier conteneur (12), des moyens étant prévus pour réaliser une liaison de transvasement entre les deux conteneurs, pour refouler le composant liquide hors du deuxième conteneur (54) dans le premier conteneur (12) et pour un mélange intimement homogène des composants dans le premier conteneur (12) par l'agitation d'un organe de brassage (30) agencé dans le premier conteneur (12) et monté sur une tige de brassage (28) creuse, guidée de manière étanche hors du conteneur, et le premier conteneur (12) étant conçu pour pouvoir être raccordé à une source de dépression, et un appareil de mesure, affichant la dépression qui règne dans le conteneur, pouvant être raccordé au premier conteneur (12),
**caractérisé en ce que** la tige de brassage (28) creuse est raccordée par son extrémité extérieure à une pompe manuelle (34), destinée à générer la dépression, et communique avec la pompe manuelle (34),
**en ce que** la tige de brassage (28) creuse, dans sa position entièrement rentrée, est munie, dans sa partie guidée de manière étanche à travers la paroi du conteneur, d'un orifice de passage (80) qui communique avec un conduit de passage (84), guidé vers l'intérieur du conteneur et muni dans cette zone d'une valve (24) à fermeture automatique, et
**en ce que** le boîtier (36) de la pompe manuelle (34) est conçu de manière appropriée sous forme d'élément d'actionnement destiné à agiter la tige de brassage (28).

2. Dispositif selon la revendication 1, **caractérisé en ce que**, pour relier le premier conteneur (12) avec le deuxième conteneur (54), il est prévu un cylindre de perforation (14), qui est hermétiquement fermé par une lamelle (18) à son extrémité orientée vers le premier conteneur (12) et qui peut être introduit et être fixé dans un logement du premier conteneur (12) et dans lequel peut être introduite une tubulure (56) s'avançant en saillie sur le deuxième conteneur (54) en étant hermétiquement fermée par une membrane (58), **en ce qu'**un corps (48) en forme de disque est agencé à l'intérieur du cylindre de perforation (14) de manière mobile dans le sens longitudinal du cylindre de perforation (14) et est traversé par une canule de perforation (50) creuse en saillie sur les deux côtés et biseautée au niveau de ses deux extrémités, et **en ce que** la longueur de la tubulure (56) en saillie sur le deuxième conteneur (54) est dimensionnée de telle sorte que celle-ci au moment de l'introduction dans le cylindre de perforation (14), fixé dans le logement du premier conteneur (12), déplace le corps (48) en forme de disque en direction du premier conteneur (12), moyennant quoi la canule de perforation (50) transperce la lamelle (18) fermant le cylindre de perforation (14), de même que la membrane (38) obturant la tubulure (56) du deuxième conteneur (54).

3. Dispositif selon la revendication 2, **caractérisé en ce que** le deuxième conteneur (54) est hermétiquement fermé à son extrémité opposée à la tubulure (56) par une membrane (60), contre laquelle est agencé un piston (62), adjacent à l'espace intérieur du conteneur et mobile de manière étanche sur la paroi intérieure du conteneur (54), et **en ce que** le deuxième conteneur (54) est maintenu de manière mobile dans un godet de guidage (70), sur le fond fermé duquel s'élèvent des tiges de poussée (72), qui sont taillées en pointe au niveau de leurs extrémités libres (74) et qui, au moment du déplacement du godet de guidage (70) en direction de la tubulure (56) du deuxième conteneur (54), transpercent la membrane (60), obturant hermétiquement le fond du deuxième conteneur et déplacent le piston (62) adjacent en direction de la tubulure (56).

4. Dispositif selon l'une quelconque des revendications 1 à 3, **caractérisé en ce que** la tige de brassage (28), guidée de manière étanche et mobile à travers un capuchon de fermeture (16) obturant le premier conteneur (12), comporte une zone de rupture théorique (36), laquelle est agencée en dehors du conteneur dans la zone de capuchon de fermeture (16) lorsque la tige de brassage (28) est dans la position entièrement extraite du premier conteneur.

5. Dispositif selon la revendication 4, **caractérisé en ce que** le capuchon de fermeture (16) est traversé par un piston (20) mobile, qui est en appui étanche contre la paroi intérieure du premier conteneur (12) et dont la face frontale, opposée à l'intérieur du conteneur, ferme à peu près en surface affleurée le capuchon de fermeture (16) dans la position extraite, mais par l'application d'une force dirigée vers l'intérieur du conteneur, est mobile en direction du logement pour le cylindre de perforation (14).

6. Dispositif selon l'une quelconque des revendications 1 à 5, **caractérisé en ce que** la pompe manuelle (34), destinée à générer la dépression dans le premier conteneur (12) et agencée à l'extrémité extérieure de la tige de brassage (28), est conçue sous forme de pompe à piston avec un piston (44), qui est agencé dans le boîtier (36) cylindrique et qui est mobile dans le boîtier au moyen d'une tige (40), qui est guidée hors du boîtier en passant par la paroi frontale opposée au premier conteneur (12) et qui comporte une poignée (42) prévue sur son extrémité libre.

7. Dispositif selon la revendication 6, **caractérisé en ce que**, dans la paroi frontale du boîtier de pompe (36), reliée à la tige de brassage (28), il est prévu une valve (46) se fermant automatiquement lorsqu'une dépression est générée du coté du conteneur dans la pompe manuelle par le déplacement du piston (44) de la pompe, et s'ouvrant sous l'effet de la formation d'une surpression.

8. Dispositif selon l'une quelconque des revendications 2 à 7, **caractérisé en ce que** le logement pour le cylindre de perforation (14) comporte un taraudage et le cylindre de perforation (14) comporte un filetage complémentaire.

9. Dispositif selon la revendication 8, **caractérisé en ce que** le cylindre de perforation (14) comporte une manette (52) ayant un diamètre plus grand.
